# EUROPÄISCHE PATENTSCHRIFT

(11) **EP 2 046 237 B1**
(45) Veröffentlichungstag und Bekanntmachung des Hinweises auf die Patenterteilung: **22.05.2013**
(21) Anmeldenummer: 07786236.5
(22) Anmeldetag: 20.07.2007
(51) Int. Cl.: A61C 8/00, A61K 6/06, A61L 27/30

(54) **IMPLANTAT ZUR VERANKERUNG VON ZAHNERSATZ**
IMPLANT FOR ANCHORING TOOTH REPLACEMENT
IMPLANT POUR L'ANCRAGE D'UNE PROTHÈSE DENTAIRE

(30) Priorität: 20.07.2006 DE 102006034092; 11.11.2006 DE 102006053260
(43) Veröffentlichungstag der Anmeldung: 15.04.2009
(73) Patentinhaber: MDI Dental- und Implantattechnik GmbH, 47057 Duisburg (DE)
(72) Erfinder: GIESELMANN, Dirk-Rolf, 8001 Zürich (CH); CESCHINSKI, Harald, 44789 Bochum (DE)
(74) Vertreter: Thiel, Christian
(86) Internationale Anmeldenummer: PCT/EP2007/006488
(87) Internationale Veröffentlichungsnummer: WO 2008/009474

(56) Entgegenhaltungen:
- EP-A- 1 334 737
- EP-A- 1 527 790
- EP-A- 1 566 152
- WO-A-2005/055860
- DE-U1-202006 012 902
- US-A- 6 165 925

## Beschreibung

Die Erfindung betrifft ein Implantat insbesondere zur Verankerung von Zahnersatz.

Implantate werden den modernen Medizin weithin eingesetzt, um angeborene oder erworbene körperliche Defekte auszugleichen. Sie finden beispielsweise in der Orthopädie Einsatz, um Frakturen zu fixieren, dienen in Form künstlicher Gelenke oder Gelenkteile dazu, Verschleißerscheinungen des Bewegungsapparates auszugleichen und finden vielfach Einsatz in der Kardiologie und im vaskulären System. Weitere Einsatzfelder sind die Stabilisierung und der Ausgleich von Schäden im Bereich des Rückgrats sowie bei Osteosynthese und Traumatologie. Schließlich spielen Implantate als Knochenersatz eine Rolle wie auch bei der Verankerung von Zahnersatz im dentalen Bereich.

Am Implantationsort kommen die Implantate unvermeidlich mit körpereigenem Gewebe in Kontakt. Dies kann beispielsweise Knochengewebe sein oder auch Weichgewebe, wie Muskelgewebe, Bindegewebe, Epithelgewebe oder auch Schleimhautgewebe. Es ist nicht nur erwünscht, dass das Implantat mit dem umgrenzenden Gewebe kompatibel ist sondern auch, dass es in dieses Gewebe einheilt und einwächst. Dabei hat das Implantatmaterial häufig nicht die gleiche Kompatibilität mit allen angrenzenden Gewebetypen.

Bei Verlust eines oder mehrerer natürlicher Zähne werden heute vielfach Zahnimplantate eingesetzt, die eine Verankerungsmöglichkeit für Zahnersatz wie Kronen, Brücken oder auch Vollprothesen darstellen.

Zahnimplantate sind in diesem Sinne im Kieferknochen verankerte Stützpfeiler, die die Funktion einer künstlichen Zahnwurzel haben. Es handelt sich um festsitzende Pfosten, die in den entsprechenden Kieferknochen eingesetzt (implantiert) werden. Sie ermöglichen sowohl das Einsetzen einzelner Zähne als auch von Brücken, ohne dabei gesunde Nachbarzähne bearbeiten zu müssen.

Bei der Implantation werden Zahnimplantate fest in einer aufgearbeiteten Zahnwurzelkavität im Kieferknochen durch Einpressen oder Einschrauben verankert. Aus dem Stand der Technik sind zwei Prinzipien der Einheilung bekannt, nämlich die "offene" und "geschlossene" Einheilung. Bei der "offenen" Einheilung ragt das sichtbare obere Ende des Implantates aus dem Kieferknochen hervor. Dagegen endet bei der "geschlossenen" Einheilung das Implantat auf Knochenniveau. Während des Heilungsprozesses, welcher in der Regel drei bis sechs Monate dauert, kommt es zum unmittelbaren Anwachsen des Knochengewebes an das Implantat. Dieses Phänomen wird mit dem Begriff Osseointegration bezeichnet.

Implantate für die "offene" Einheilung weisen einen enossalen und einen gingivalen Teil auf. Der enossale Teil befindet sich im Kieferknochen. Der gingivale Teil ragt aus dem Kieferknochen heraus in das Zahnfleisch bzw. die Gingiva. Während der enossale Teil der Osseointegration unterliegt, lagert sich an den gingivalen Teil Bindegewebe und Epithelgewebe an.

Bei Implantaten aus Titan, einem vielfach für Zahnimplantate verwandten Material, kommt es bei der Osseointegration selten zu Komplikationen. Titan und Knochengewebe sind gut miteinander verträglich und die Osseointegration schreitet in der Regel rasch und problemlos voran.

Anders sieht es bei der Verträglichkeit von Titanmetall mit Epithelgewebe und gingivalem Bindegewebe aus. Hier ist die Verträglichkeit deutlich geringer, und der Ein- und Anheilungsprozess dauert häufig länger als erwünscht. Als Komplikationen sind Periimplantitis und Plaqueakkumulationen bekannt. Tatsächlich sollte die Einheilung des gingivaten Teils des Implantates in die Gingiva nicht länger in Anspruch nehmen als die Osseointegration.

Es ist bekannt, dass Epithel- und Bindegewebe sich sehr gut mit keramischen Materialien verträgt, insbesondere mit Zirkonoxid. Die Verwendung von Zirkonoxid für Implantate ist bekannt, darunter auch für Zahnimplantate. Allerdings hat sich bei Zahnimplantaten Zirkonoxid den weit verbreiteten Titanimplantaten nicht immer als gleichwertig erwiesen. Hierfür dürfte auch die außerordentlich hohe Druckbelastung verantwortlich sein, denen Zahnimplantate beim Kauvorgang ausgesetzt sind.

Wegen der außerordentlich guten Verträglichkeit von Zirkonoxidkeramik mit Epithel- und gingivalem Bindegewebe wurde bereits vorgeschlagen, den gingivalen Teil von Zahnimplantaten mit einer Zirkonoxidmanschette zu versehen, die auf die Titanoberfläche aufgeklebt wird. auf diese Art und Weise wird erreicht, dass das gingivale Gewebe nicht mit dem Titanmetall des eigentlichen Implantates in Berührung kommt. Bei dieser Kombination aus Implantat und Manschette hat sich aber die Klebeverbindung als nicht optimal erwiesen, d. h. die Manschette neigt bereits zu einem frühen Zeitpunkt dazu, sich vom Titankern zu trennen.

Dentalimplantate, die im gingivalen Bereich eine Zirkonoxidmanschette aufweisen, sind in WO 2003/013385 A beschrieben.

Die EP 1 527 790 A1 lehrt ein Zahnimplantat gemäß dem Oberbegriff des Anspruchs 1.

Angesichts dieser Situation liegt der Erfindung die Aufgabe zugrunde, Implantate bereitzustellen, die die gute Verträglichkeit bzw. die Osseointegrationseigenschaften von Metallimplantaten weiter verbessern und die bezüglich des Ein- und Anwachsens von Weichgewebe und in ihrer Dauerhaftigkeit optimiert sind.

Diese Aufgabe wird mit Implantaten der eingangs genannten Art gelöst, die eine Beschichtung aus stabilisiertem Zirkonnitrid und/oder Zirkonoxid aufweisen.

Die erfindungsgemäßen Implantate können in nahezu allen medizinischen Bereichen eingesetzt werden. Sie werden nachstehend anhand von Zahnimplantaten näher beschrieben. Entsprechend ist bei den Zahnimplantaten das Weichgewebe, das mit den Implantaten in Kontakt kommt, die Gingiva, worauf der gingivale Abschnitt des Implantates Bezug nimmt. Bei anderen Formen von Implantaten kann dieses Weichgewebe, Bindegewebe, Muskelgewebe, Epithelgewebe oder auch eine Form von mucosalem Gewebe sein.

Bei den Zahnimplantaten vermeidet die Beschichtung des gingivalen Abschnitts die Notwendigkeit einer Klebstoffschicht, wie sie mit der Manschette gemäß WO 2003/013385 erforderlich ist.

Metalle können mit guter Haftung innerhalb der Schicht und von der Schicht zum Metall beschichtet werden, insbesondere durch Kathodenzerstäubung. Bei der Kathodenzerstäubung wird Material von einer Kathode (Target) abgetragen und auf einem Substrat niedergeschlagen. Die Beschichtung erfolgt in einer Vakuumkammer in Gegenwart eines Inertgases wie Argon (Gasfluss-Sputtern, GFS). Beim reaktiven Sputtern kann ein weiteres Gas eingespeist werden, das mit den aus dem Target herausgelösten Ionen reagiert. Die daraus resultierenden Schichten sind keramischer Natur, wenn Sauerstoff oder Stickstoff eingespeist werden.

Vorzugsweise wird bei der Erfindung zur Beschichtung des gingivalen Abschnitts des Implantates von solchem reaktiven Gasflusssputtern in einer Vakuumkammer in Gegenwart von Stickstoff oder Sauerstoff Gebrauch gemacht. Diesbezüglich wird auf die DE 199 58 643 C1 und die darin beschriebenen Techniken zur Beschichtung von Turbinenschaufeln mit Zirkonoxid hingewiesen. Als Target dient metallisches Zirkonium, das mit bis zu 10 Gew.-% Yttrium legiert ist. Das oder mehrere Implantate befinden sich auf einem beheizten Substrathalter.

Um zu verhindern, dass sich die Beschichtung auch auf dem enossalen Abschnitt des Implantates niederschlägt, wird dieser auf an und für sich bekannte Weise abgeschirmt. Entsprechendes gilt für andere nur teilweise zu beschichtende Implantate.

Dem Zirkonoxid kann in bekannter Weise ein Metalloxid beigemischt werden. Solches polykristallines "stabilisiertes" Zirkonoxid enthält beispielsweise bis zu 10 Gew.-% Yttriumoxid (Y₂O₃), gegebenenfalls auch Aluminiumoxid (Al₂O₃), Magnesiumoxid (MgO), Calziumoxid (CaO) oder auch mehrere davon. Die Schicht aus stabilisiertem Zirkonoxid kann beispielsweise durch gleichzeitige Kathodenzerstäubung von Zirkonium und Yttrium in Gegenwart von Sauerstoff erzeugt werden. Wenn im folgenden von "Zirkonium" die Rede ist, kann dieses als Metall wie als Nitrid oder Oxid bis zu 10 Gew.-% Yttrium aufweisen, in der Regel 5 bis 10 Gew.-%.

Unabhängig davon sind auch andere Sputterverfahren zur Herstellung der gewünschten Beschichtung geeignet, beispielsweise DC-Sputtern, HF-Sputtern, Magnetronsputtern und Ionenstrahlsputtern, auch durch Elektronenstrahlbedampfen. Das GFS-Verfahren ist bevorzugt.

Eine Zirkonnitrid-Beschichtung des Implantates hat eine gelbe bis goldgelbe Farbe, die geeignet ist, die Grau-Färbung des Metalls in erwünschter Weise schon in dünnen Schichten zu überdecken. Eine solche Zirkonnitridschicht hat in der Regel eine Schichtdicke von 1 bis 10 µm, je nach gewünschter Farbtiefe und Dicke der außerordentlich harten Zirkonnitridschicht.

Physiologisch gesehen hat Zirkonnitrid den Vorteil nicht nur einer außerordentlich hohen Härte, die die Abrasionsbeständigkeit der Beschichtung erhöht, sondern auch außerordentlich guter physiologischer Verträglichkeit. Zirkonnitrid fördern wie Zirkonoxid das Anwachsen von gingivalem Gewebe. Hinzu kommt, dass Zirkonnitrid wie Zirkonoxid die bei der Metallerarbeitung entstandenen Bearbeitungsstrukturen aufnehmen und an die Oberfläche weitergeben, d. h. die Implantate haben auch in beschichteter Form eine quer verlaufende Riffelung, die in der Oberfläche aufscheint. Die Besiedelung mit gingivalen Zellen verläuft bevorzugt in diesen Riffelungen und damit quer zur Hauptbelastungsrichtung des Implantates, was zusätzliche Festigkeit verleiht.

Zur weiteren Verbesserung der Haftung der Beschichtung auf dem Metall des Implantates kann es zweckmäßig sein, eine Grundierung und/oder Zwischenschicht anzulegen, beispielsweise durch Oxidation der Metalloberfläche (Titan zu Titandioxid), durch Aufbringen einer Grundschicht aus Zirkonium oder durch Aufbringen einer Zwischenschicht aus Zirkonnitrid oder Aluminiumoxid (Al₂O₃). Auch diese Grundschicht oder Zwischenschichten können durch Kathodenzerstäubung erstellt werden.

Beispielsweise kann auf die im GFS-Verfahren gereinigte Titanoberfläche zunächst eine Grundschicht aus Zirkonium aufgebracht werden, beispielsweise in einer Schichtdicke von 0,5 bis 2 µm. Anschließend wird eine Zwischenschicht aus Zirkonnitrid (ZrN) aufgebracht, die eine Schichtdicke von 1 bis 10 µm haben kann. Auf diese wird dann die Deckschicht aus Zirkonoxid aufgebracht. Alle Beschichtungen können in einem Arbeitsgang durchgeführt werden, wobei die Reinigung durch Beglimmen in Gegenwart von Argon, die Grundschicht durch Besputtern mit Zirkonium als Target in Gegenwart von Argon, die Nitridschicht durch Besputtern mit Zirkonium als Target in Gegenwart von Argon und Stickstoff und die Oxidschicht durch Besputtern mit Zirkonium als Target in Gegenwart von Argon und Sauerstoff vorgenommen wird. Das Substrat wird auf eine erhöhte Temperatur von bis zu 400°C gebracht. Die Biasspannung liegt bei bis zu 200V, je nach Verfahrensschritt, die Biasfrequenz bei bis zu 200 kHz, ebenfalls ja nach Verfahrensschritt.

Im Allgemeinen hat die Zirkonoxidbeschichtung eine Schichtdicke von 2 bis 50 µm, insbesondere 5 bis 15 µm. Bei der Grundierung reicht - falls erforderlich - im Allgemeinen eine Schichtdicke von etwa 1 µm aus.

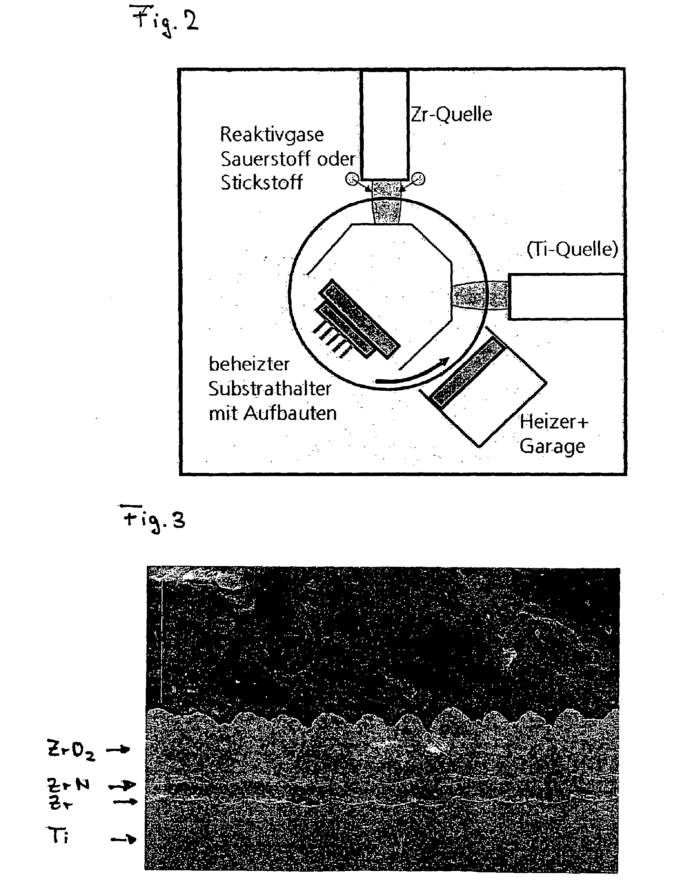

Ein typisches Zahnimplantat, wie in Figur 3 dargestellt, hat beispielsweise auf der gereinigten Titanoberfläche eine Schichtdicke von 0,5 bis 2 µm Zirkonium, insbesondere etwa 1 bis 1,5 µm, 1 bis 10 µm Zirkonnitrid, insbesondere etwa 4 bis 5 µm und 10 bis 50 µm Zirkonoxid, insbesondere 10 bis 20 µm und besonders bevorzugt etwa 15 µm.

Eine derart aufgebaute Beschichtung ergibt eine hinreichend harte Oberfläche mit einer akzeptablen Farbgebung und exzellenten Einheilungseigenschaften, wobei die Affinität des mukosalen Gewebes für die Zirkonoxidoberfläche der bei der Zahnprothetik gefürchteten Parodontose entgegenwirkt.

Die Beschichtung des Implantates umfasst im Allgemeinen dessen gingivalen Abschnitt, d. h. alle Teile, die mit der Gingiva in Kontakt kommen. Die Beschichtung kann sich entsprechend auch auf die Kopfseite des Implantates ausdehnen und gegebenenfalls auf Teile, die aus der Gingiva heraus in den Mundraum ragen und zur Festlegung beispielsweise der Krone dienen.

Bei Implantaten, die aus mehreren Teilen zusammengesetzt sind, beispielsweise den eingangs erwähnten Implantaten für die "geschlossene" Einheilung, die mit dem Knochenniveau enden, versteht es sich, dass der in diesen Teil des Implantates eingesetzte gingivale Aufsatz entsprechend mit stabilisiertem Zirkonoxid beschichtet ist. Beschichtet sein können ferner Stifte oder Kopfteile, die auf die Implantate aufgesetzt werden, um die eigentliche Prothetik festzuhalten. Die Beschichtung derartiger Stifte oder Köpfe hat den Vorteil, dass eine Verbesserung der Optik herbeigeführt wird. Dadurch dass die aufgesetzten Kronen zumeist transluzent sind, neigt der Metallkern dazu, nach außen durchzuscheinen. Dies gibt einen optisch unvorteilhaften dunklen Schatten in der Zahnprothetik. Die Beschichtung dieser Stifte oder Kopfteile führt zu einer optischen Abschirmung und damit zu einer Verbesserung der Optik der gesamten Zahnprothetik.

Darüber hinaus haben die erfindungsgemäßen Implantate weitere Vorteile. So vermeiden oder vermindern sie die bei zahlreichen Zahnimplantaten auftretende Periimplantitis, die letztlich auch wieder zum Verlust des Implantates führen kann. Des Weiteren geht damit auch eine Verringerung der Plaqueakkumulation einher.

Entsprechendes gilt für Implantate, die für den nicht-dentalen Bereich der Medizin bestimmt sind. Auch hier können die Implantate ganz oder teilweise beschichtet sein, wobei die Beschichtung insbesondere die Teile betrifft, die mit Weichgewebe in Berührung kommen.

Die erfindungsgemäß beschichteten Implantate sind in ihrem Einwachsverhalten insbesondere in das Weichgewebe optimiert und haben eine deutlich bessere Gewebeverträglichkeit. Dies trägt dazu bei, die Einheilungszeit zu verkürzen und die Belastbarkeit gegenüber herkömmlichen Implantaten zu erhöhen.

Die Erfindung wird durch die beiliegenden Abbildungen näher erläutert.

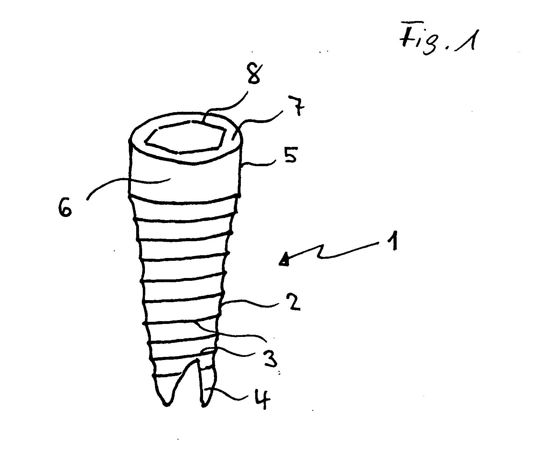

Abbildung 1 zeigt ein erfindungsgemäßes Implantat 1, das mit seinem enossalen Abschnitt 2 über das Gewinde 3 in den Kieferknochen eingeschraubt wird. Das Wurzelteil 4 ist in üblicher Weise mit Einschnitten versehen, die die Osseointegration erleichtert.

Der gingivale Abschnitt 5, der sich an den enossalen Abschnitt 2 anschließt, ist mit einer dünnen Schicht einer Zirkonoxidkeramik 6 beschichtet, die sich auf die Flanke des Implantates beschränken kann, aber auch den Kopfbereich 7 erfassen kann. Im Kopfbereich 7, der mit der Gingiva abschließt, weist eine hexagonale Öffnung auf, die zum einen dazu verwandt werden kann, das Implantat in den Kiefer einzuschrauben, zum anderen der Aufnahme eines Stiftes dient, der die eigentliche Zahnprothetik trägt.

Abbildung 2 zeigt das Schema des Beschichtungsaufbaus mit rotierendem Substratteller. Die Beschichtungskammer ist eine polygonale Vakuumanlage mit einem Kammervolumen von 200 l, die mit einer horizontal arbeitenden GFS-Linearquelle ausgestattet ist. Diese ist mit metallischen Targets aus Zirkonium-Yttrium (92,2:7,8 Gew.-%) bestückt. Bei reaktiver Prozessführung wird unter Sauerstoffzugabe Yttrium-teilstabilisiertes Zirkoniumoxid abgeschieden, bei Stickstoffzugabe wird Zirkonnitrid (+ Yttriumnitrid) abgeschieden. Eine zweite Sputterquelle (Ti-Quelle) ermöglicht die Aufbringung einer zusätzlichen Titan-Haftschicht.

Nahe der Zr-Quelle befinden sich Einlässe für die Reaktivgase Sauerstoff und Stickstoff.

Für die Aufnahme des Substrats wurde ein rückseitig beheizter Substrathalter verwendet. Ein keramischer Heizstrahler ermöglicht die Erzeugung von Substrattemperaturen bis 400°C, die mit einem Thermoelement in Kontakt mit der Halteplatte überwacht wurde.

Die Arbeitsgase sind Argon für den Materialtransport sowie Sauerstoff und Stickstoff als Reaktivgase, jeweils in hoch reiner Form mit wenigstens 99,99 %.

Für die Beschichtung werden zunächst die im Substrathalter gehaltenen Implantate in Gegenwart von Argon beglimmt, um oberflächliche

Verunreinigungen und Oxidschichten abzutragen. Im Anschluss daran wird in Gegenwart von Argon eine Zirkonium-Grundschicht aufgetragen, anschließend unter zusätzlicher Einspeisung von Stickstoff eine Zirkonnitridzwischenschicht. Im Anschluss daran kann in Gegenwart von Sauerstoff (Stickstoff wird abgeschaltet) die Zirkonoxid-Deckschicht aufgebracht.

Es versteht sich, dass zur Erzielung besonders harter Oberflächen auf die Zirkonoxid-Deckschicht verzichtet werden kann und in diesem Fall eine Deckschicht aus Zirkonnitrid aufgebracht wird. Eine solche Zirkonnitrid-Deckschicht kommt, aufgrund der außerordentlich großen Härte des Materials, mit einer Schichtdicke von etwa 10 µm aus, kann aber auch in dickeren Schichten bis zu 20 µm aufgetragen werden.

## Patentansprüche

1. Zahnimplantat zur Verankerung von Zahnersatz, **gekennzeichnet durch** eine Beschichtung (6) aus Zirkonnitrid (ZrN) mit einer Schichtdicke von 1 bis 10 µm.

2. Implantat nach Anspruch 1, **dadurch gekennzeichnet, dass** die Zirkonnitridbeschichtung (6) durch Kathodenzerstäubung aufgebracht ist.

3. Implantat nach Anspruch 2, **dadurch gekennzeichnet, dass** die Zirkonnitridbeschichtung (6) durch reaktives Gasflusssputtem in einer Vakuumkammer in Gegenwart von Stickstoff aufgebracht ist.

4. Implantat nach einem der vorstehenden Ansprüche, **dadurch gekennzeichnet, dass** es eine haftungsfördemde Grundschicht aufweist.

5. Implantat nach Anspruch 4, **dadurch gekennzeichnet, dass** die Grundschicht aus Zirkonium, Titanoxid (TiO₂) und/oder Aluminiumoxid (Al₂O₃) besteht.

6. Implantat nach einem der vorstehenden Ansprüche, **dadurch gekennzeichnet, dass** es eine harte Zwischenschicht aufweist.

7. Implantat nach einem der Ansprüche 1 bis 6, **dadurch gekennzeichnet, dass** zumindest die mit Weichgewebe (Gingiva) in Kontakt kommenden Abschnitte (5) des Implantats beschichtet sind.

8. Implantat nach Anspruch 4 oder 5, **gekennzeichnet durch** eine Grundschicht aus Zirkonium und eine Deckschicht aus Zirkonnitrid (ZrN).

9. Implantat nach einem der vorstehenden Ansprüche, **dadurch gekennzeichnet, dass** die Beschichtung (6) auf den Kopf (7) des Implantats aufgebracht ist.

10. Implantat nach einem der vorstehenden Ansprüche, **dadurch gekennzeichnet, dass** es aus Titan oder einer Titanlegierung besteht.

## Claims

1. A dental implant for anchoring dental prostheses, **characterized by** a zirconium nitride (ZrN) coating (6) having a thickness of 1 to 10 µm.

2. The implant according to claim 1, **characterized in that** the zirconium nitride coating (6) is applied by cathode sputtering.

3. The implant according to claim 2, **characterized in that** the zirconium nitride coating (6) is applied by reactive gas flow sputtering performed in a vacuum chamber in the presence of nitrogen.

4. The implant according to any of the above claims, **characterized in that** it is provided with an adherence-promoting base coating.

5. The implant according to claim 4, **characterized in that** the base coating consists of zirconium, titanium oxide (TiO₂) and/or aluminium oxide (Al₂O₃).

6. The implant according to any of the above claims, **characterized in that** it is provided with a hard intermediate coating.

7. The implant according to any of the claims 1 to 6, **characterized in that** the coating (6) covers at least the sections (5) of the implant that come into contact with soft tissue (gingiva).

8. The implant according to claim 4 or 5, **characterized by** a zirconium base coating and a zirconium nitride (ZrN) covering coating.

9. The implant according to any of the above claims, **characterized in that** the coating (6) is applied to the top part (7) of the implant.

10. The implant according to any of the above claims, **characterized in that** it consists of titanium or a titanium alloy.

## Revendications

1. Implant dentaire pour l'ancrage d'une prothèse dentaire, **caractérisé par** un revêtement (6) en nitrure de zirconium (ZrN) d'une épaisseur de couche de 1 à 10 µm.

2. Implant selon la revendication 1, **caractérisé en ce que** le revêtement en nitrure de zirconium (6) est appliqué par pulvérisation cathodique.

3. Implant selon la revendication 2, **caractérisé en ce que** le revêtement en nitrure de zirconium (6) est appliqué par pulvérisation cathodique en gaz réactif dans une chambre à vide en présence d'azote.

4. Implant selon l'une quelconque des revendications précédentes, **caractérisé en ce qu'**il comporte une couche de fond promotrice d'adhérence.

5. Implant selon la revendication 4, **caractérisé en ce que** la couche de fond consiste en zirconium, en oxyde de titane (TiO₂) et/ou en oxyde d' aluminium (Al₂O₃).

6. Implant selon l'une quelconque des revendications précédentes, **caractérisé en ce qu'**il comporte une couche intermédiaire dure.

7. Implant selon l'une quelconque des revendications 1 à 6, **caractérisé en ce qu'**au moins les sections (5) de l'implant entrant en contact avec des tissus mous (gencive) sont revêtues.

8. Implant selon la revendication 4 ou 5, **caractérisé par** une couche de fond en zirconium et une couche de couverture en nitrure de zirconium (ZrN).

9. Implant selon l'une quelconque des revendications précédentes, **caractérisé en ce que** le revêtement (6) est appliqué sur la tête (7) de l'implant.

10. Implant selon l'une quelconque des revendications précédentes, **caractérisé en ce qu'**il est en titane ou en un alliage de titane.
